# EUROPÄISCHE PATENTSCHRIFT

(11) **EP 2 609 939 B1**
(45) Veröffentlichungstag und Bekanntmachung des Hinweises auf die Patenterteilung: **29.10.2014**
(21) Anmeldenummer: 13158897.2
(22) Anmeldetag: 17.05.2010
(51) Int. Cl.: A61L 15/60, A61F 13/53, A61L 15/42, A61F 13/532

(54) **Wasserabsorbierende Speicherschichten**
Water-absorbent storage layers
Couches de rétention hydroabsorbantes

(30) Priorität: 20.05.2009 EP 09160762
(43) Veröffentlichungstag der Anmeldung: 03.07.2013
(62) Teilanmeldung aus: 10719351.8
(73) Patentinhaber: BASF SE, 67056 Ludwigshafen (DE)
(72) Erfinder: Bauduin, Christophe, 68723 Plankstadt (DE)

(56) Entgegenhaltungen:
- EP-A1- 1 504 772
- WO-A2-02/094328
- US-A1- 2003 135 172

## Beschreibung

Die vorliegende Erfindung betrifft verbesserte wasserabsorbierende Speicherschichten für den Einsatz in Hygieneartikeln, wobei die wasserabsorbierenden Speicherschichten im Wesentlichen frei von Zellulosefasern sind.

Die Herstellung wasserabsorbierender Polymerpartikel und deren Verwendung zur Herstellung von Hygieneartikeln wird beispielsweise in der Monographie "Modern Superabsorbent Polymer Technology". F.L. Buchholz und A.T. Graham, Wiley-VCH, 1998, insbesondere auf den Seiten 252 bis 258, beschrieben. Die wasserabsorbierenden Polymerpartikel werden auch als Superabsorber bezeichnet.

Die zurzeit kommerziell verfügbaren Einwegwindeln bestehen üblicherweise aus einer flüssigkeitsdurchlässigen oberen Schicht (A), einer flüssigkeitsundurchlässigen unteren Schicht (B), einer wasserabsorbierenden Speicherschicht (C) zwischen den Schichten (A) und (B) und einer Aufnahme- und Verteilschicht (D) zwischen den Schichten (A) und (C),

Die wasserabsorbierende Speicherschicht besteht üblicherweise aus einem Gemisch aus wasserabsorbierenden Polymerpartikeln und Zellulosefasern, wobei die wasserabsorbierenden Polymerpartikel durch die Zellulosematrix fixiert sind.

In den letzten Jahren ging der Trend zu immer dünneren Einwegwindeln. Zur Herstellung immer dünnerer Einwegwindeln wurde der Anteil an Zellulosefasern in der wasserabsorbierenden Speicherschicht immer mehr gesenkt. Nachteilig hierbei ist, dass die Zellulosematrix dadurch immer weiter ausgedünnt wird und die Beweglichkeit der wasserabsorbierenden Polymerpartikel in der wasserabsorbierenden Speicherschicht zunimmt.

Insbesondere, wenn wasserabsorbierende Speicherschichten gewünscht werden, die im Wesentlichen frei von Zellulosefasern sind, d.h. nahezu ausschließlich aus wasserabsorbierenden Polymerpartikeln bestehen, besteht die Gefahr, dass die wasserabsorbierenden Polymerpartikel innerhalb der Einwegwindel verrutschen oder sogar ganz aus der Einwegwindel herausfallen.

Zu Lösung dieses Problems wurden neuartige wasserabsorbierende Speicherschichten hergestellt. Beispielsweise beschreibt WO 97/17397 A1 ein Verfahren zur Herstellung wasserabsorbierender Schäume. Bei Verwendung derartiger Schäume kann auf den Einsatz von Zellulosefasern vollständig verzichtet werden.

Zellulosefreie Hygieneartikel können auch durch Fixierung von wasserabsorbierenden Polymerpartikeln mittels thermoplastischer Polymere, insbesondere von Schmelzklebern, auf geeigneten Vliesunterlagen befestigt werden, sofern man diese thermoplastischen Polymere zu feinen Fasern ausspinnt. Solche Produkte werden beispielsweise in US 2003/0181115, US 2004/0167486, US 2004/071363, US 2005/097025, US 2007/156108, US 2008/0125735, EP 1 917 940 A1, EP 1 913 912 A1, EP 1 913 913 A2, EP 1 913 914 A2, EP 1 911 425 A2, EP 1 911 426 A2, EP 1 447 067 A1, EP 1 813 237 A2, EP 1 813 236 A2, EP 1 808 152 A2, EP 1 447 066 A1 beschrieben. Die Herstellverfahren sind in den WO 2008/155722 A2, WO 2008/155702 A1, WO 2008/155711 A1, WO 2008/155710 A1, WO 2008/155701 A2, WO 2008/155699 A1 offenbart. Nachteilig ist das relativ komplexe Herstellverfahren, da das Ausspinnen der Klebstofffasern in Gegenwart von wasserabsorbierenden Polymerpartikeln schwierig und fehleranfällig ist.

Weiterhin sind dehnbare zellulosefreie Hygieneartikel bekannt und in den US 2006/0004336, US 2007/0135785, US 2005/0137085 ist deren Herstellung durch gleichzeitiges Faserspinnen von geeigneten thermoplastischen Polymeren und Einarbeitung von wasserabsorbierenden Polymerpartikeln offenbart. Auch dieses Verfahren ist komplex und fehleranfällig.

Aufgabe der vorliegenden Erfindung war die Bereitstellung verbesserter wasserabsorbierender Speicherschichten für Hygieneartikel, insbesondere Einwegwindeln. Für die verbesserten wasserabsorbierenden Speicherschichten sollten die üblichen wasserabsorbierenden Polymerpartikel verwendbar sein. Weiterhin sollten die verbesserten wasserabsorbierenden Speicherschichten im Wesentlichen frei von Zellulosefasern sein und die wasserabsorbierenden Polymerpartikel in der wasserabsorbierenden Speicherschicht sowohl im trockenen als auch im feuchten Zustand weder verrutschen noch herausfallen. Frei von Zellulosefasern bedeutet im Sinne dieser Anmeldung, dass der Zelluloseanteil in der erfindungsgemäßen Speicherschicht vorzugsweise weniger als 30 Gew.-%, bevorzugt weniger als 20 Gew.-%, besonders bevorzugt weniger als 10 Gew.-%, ganz besonders bevorzugt weniger als 5 Gew.-%, beträgt. Im Idealfall ist gar keine Zellulose enthalten.

Gelöst wurde die Aufgabe durch wasserabsorbierende Speicherschichten, bestehend aus einer Vliesunterlage, wasserabsorbierenden Polymerpartikeln und einer flüssigkeitsdurchlässigen Abdeckung, dadurch gekennzeichnet, dass die wasserabsorbierenden Polymerpartikel auf der Vliesunterlage fixiert sind, wie definiert in Anspruch 1.

In der vorliegenden Erfindung wird eine weiche Matrix aus einem flüssigkeitsdurchlässigen Material auf die Vliesunterlage aufgebracht und die wasserabsorbierenden Polymerpartikel in die Kammern der Matrix eingebracht. Die Kammern der Matrix werden mit einer flüssigkeitsdurchlässigen Abdeckung verschlossen. Die weiche Matrix wird vorzugsweise mit der Vliesunterlage und der flüssigkeitsdurchlässigen Abdeckung verklebt.

Vorteilhaft bei dieser Ausführungsform ist, dass das Matrixmaterial so ausgewählt werden kann, dass es zusätzlich die Flüssigkeitsverteilung innerhalb der wasserabsorbierenden Speicherschicht unterstützt. Geeignet sind hierfür verpresste hydrophile Fasern (beispielsweise aus Zellulose, chemisch gefällter Zellulose oder vernetzter Zellulose) oder offenporige weiche Schwämme. Im Falle von Schwämmen sind hydrophile Typen bevorzugt. Das Matrixmaterial soll im expandierten Zustand (ungepresst) durchgehende Poren mit vorzugsweise von 0,001 bis 2,0 mm, bevorzugt von 0,01 bis 1,0 mm, besonders bevorzugt von 0,03 bis 0,5 mm, ganz besonders bevorzugt von 0,06 bis 0,3 mm, Durchmesser aufweisen.

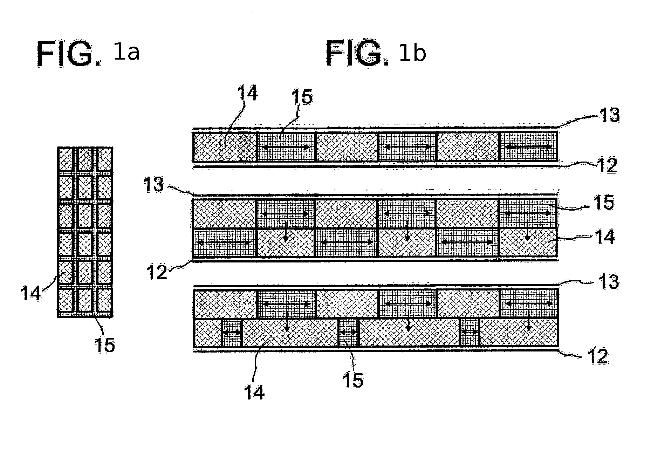

Die Figur 1a zeigt eine Draufsicht und die Figur 1b zeigt Querschnitte der erfindungsgemäßen wasserabsorbierenden Speicherschichten, wobei die Bezugszeichen die folgenden Bedeutungen haben:
- 12: Vliesunterlage
- 13: flüssigkeitsdurchlässige Abdeckung
- 14: wasserabsorbierende Polymerpartikel
- 15: flüssigkeitsdurchlässige Matrix

In allen Ausführungsformen kann in einer weiteren besonders bevorzugten Variante zusätzlich ein wasserlöslicher Kleber zur Trockenfixierung der wasserabsorbierenden Polymerpartikel verwendet werden. Der Kleber wird beispielsweise auf die Vliesunterlage vor dem Aufbringen der wasserabsorbierenden Polymerpartikel aufgebracht. Die Auftragung kann beispielsweise punktuell, flächig, oder bevorzugt in Streifen in oder quer oder diagonal zur Maschinenlaufrichtung erfolgen. Der wasserlösliche Kleber kann beispielsweise aus Polyvinylalkohol, Polyvinylpyrrolidon, Polyethylenglykol, Stärke und Stärkederivaten, Zellulose und Zellulosederivaten oder Polyacrylsäure bestehen. Ganz besonders bevorzugt enthält der wasserlösliche Kleber mindestens ein Polyamin oder besteht daraus. Geeignete Polyamine sind Polyvinylamine, Polyethylenimine, Polyallylamine. Besonders bevorzugt ist Polyvinylamin. Bei Kontakt mit Feuchtigkeit löst sich das Amin aus dem Kleber und zieht auf das quellende Hydrogel auf, wodurch es zusätzlich zu einer besonderen Gelschichtstabilität im gequollenen Zustand kommt.

In bevorzugten Ausführungsformen wird eine Bahn der Vliesunterlage in Maschinenlaufrichtung bewegt und darauf werden Streifen oder geometrische Muster mit wasserabsorbierenden Polymerpartikeln aufgebracht. In der zweiten Ausführungsform der vorliegenden Erfindung kann dabei eine geschlossene Fläche erhalten werden. Bei allen drei Ausführungsformen ist aber auch beliebige geometrische Formen und Muster denkbar, beispielsweise eine die wie Kissen mit wasserabsorbierenden Polymerpartikeln in der Fläche angeordnet sind. Die Kissen bzw. die Haufen der aufgebrachten wasserabsorbierenden Polymerpartikel können dabei jede beliebige Form in der Fläche annehmen wie beispielsweise Kreise, Ellipsen, Rechtecke, Quadrate, Dreiecke (von oben gesehen). Besonders bevorzugt sind beliebige Polygone oder Mischungen von Polygonen mit denen sich die zweidimensionale Fläche lückenlos belegen lässt. Besonders bevorzugt ist auch das Aufbringen einer oder mehrerer durchgehender Streifen in Maschinenlaufrichtung wobei die Streifen parallel zueinander verlaufen.

Im Fall von Taschen ist es vorteilhaft diese lose zu füllen damit die wasserabsorbierenden Polymerpartikeln möglichst unbehindert quellen können.
Wahlweise kann aber auch ein elastisches Vlies zur Abdeckung oder als Unterlage verwendet werden. Solche Vliese sind kommerziell erhältlich.

In allen Ausführungsformen wird die die Vliesunterlage mittels Unterdruck auf einer geeigneten Maschine so fixiert, dass abzulegende wasserabsorbierende Polymerpartikel dann mittels Masken oder ähnlichen Mitteln dort abgelegt werden können, so dass diese wasserabsorbierende Polymerpartikel durch den herrschenden Sog während der Verarbeitung von unten fixiert gehalten werden. Ebenso kann man so die anderen Komponenten temporär fixieren.

### A. Wasserabsorbierende Polymerpartikel

Die wasserabsorbierenden Polymerpartikel werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Monomere a) enthalten üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraalloxyethan, Methylenbismethacrylamid, 15-fach ethoxiliertes Trimethylolpropantriacrylat, Polyethylenglykoldiacrylat, Trimethylolpropantriacrylat und Triallylamin.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils bezogen auf Monomer a). Mit steigendem Vernetzergehalt sinkt die Zentrifugenretentionskapazität (CRC) und die Absorption unter einem Druck von 21,0 g/cm² durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite® FF6 und Brüggolite® FF7 (Brüggemann Chemicals; Heilbronn; DE) erhältlich.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2 und WO 2008/052971 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA (European Disposables and Nonwovens Association) empfohlenen Testmethode Nr. WSP 230.2-05 "Moisture Content" bestimmt wird. Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA (European Disposables and Nonwovens Association) empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Flüssigkeitsweiterleitung oder Permeabilität (SFC oder GBP). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr früh zugesetzt, beispielsweise bereits zur Monomerlösung, so wird dadurch die Zentrifugenretentionskapazität (CRC) der erhaltenen wasserabsorbierenden Polymerpartikel gesenkt. Dies kann aber beispielsweise durch Anpassung der Einsatzmenge an Vernetzer b) kompensiert werden.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyloxazolidin-2-on, Oxazolidin-2-on und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Obertlächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Tartrat, Citrat und Lactat, möglich. Aluminiumsulfat, basisches Aluminiumacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; DE), Vrieco-Nauta Continuous Mixer (Hosokawa Micron BV; Doetinchem; NL), Processall Mixmill Mixer (Processall Incorporated; Cincinnati; US) und Schugi Flexomix® (Hosokawa Micron BV; Doetinchem; NL). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Wird ausschließlich Wasser als Lösungsmittel verwendet, so wird vorteilhaft ein Tensid zugesetzt. Dadurch wird das Benetzungsverhalten verbessert und die Verklumpungsneigung vermindert. Vorzugsweise werden aber Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; DE), Hosokawa Bepex® Disc Dryer (Hosokawa Micron GmbH; Leingarten; DE) und Nara Paddle Dryer (NARA Machinery Europe; Frechen; DE). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die optionale Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert.

Geeignete Beschichtungen zur Verbesserung der Anquellgeschwindigkeit sowie der Flüssigkeitsweiterleitung oder Permeabilität (SFC oder GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Die gemäß dem erfindungsgemäßen Verfahren hergestellten wasserabsorbierenden Polymerpartikel weisen einen Feuchtegehalt von vorzugsweise 0 bis 15 Gew.-%, besonders bevorzugt 0,2 bis 10 Gew.-%, ganz besonders bevorzugt 0,5 bis 8 Gew.-%, auf, wobei der Feuchtegehalt gemäß der von der EDANA (European Disposables and Nonwovens Association) empfohlenen Testmethode Nr. WSP 230.2-05 "Moisture Content" bestimmt wird.

Die gemäß dem erfindungsgemäßen Verfahren hergestellten wasserabsorbierenden Polymerpartikel weisen eine Zentrifugenretentionskapazität (CRC) von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. WSP 241.2-05 "Centrifuge Retention Capacity" bestimmt.

Die gemäß dem erfindungsgemäßen Verfahren hergestellten wasserabsorbierenden Polymerpartikel weisen eine Absorption unter einem Druck von 49,2 g/cm² von typischerweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Die Absorption unter einem Druck von 49,2 g/cm² der wasserabsorbierenden Polymerpartikel beträgt üblicherweise weniger als 35 g/g. Die Absorption unter einem Druck von 49,2 g/cm² wird analog der von der EDANA (European Disposables and Nonwovens Association) empfohlenen Testmethode Nr. WSP 242.2-05 "Absorption under Pressure" bestimmt, wobei statt eines Drucks von 21,0 g/cm² ein Druck von 49,2 g/cm² eingestellt wird.

### B. Hygieneartikel

Die Hygieneartikel, insbesondere Einwegwindeln, bestehen aus
(A) einer flüssigkeitsdurchlässigen oberen Schicht (upper liquid-pervious layer),
(B) einer flüssigkeitsundurchlässigen unteren Schicht (lower liquidimpervious layer),
(C) einer wasserabsorbierenden Speicherschicht (water-absorbent core) zwischen Schicht (A) und Schicht (B) und
(D) optional einer Aufnahme- und Verteilschicht (acquisition distribution layer) zwischen Schicht (A) und Schicht (C).

Bei der flüssigkeitsdurchlässigen oberen Schicht (A) handelt es sich um die Schicht, die direkten Hautkontakt hat. Das Material hierfür besteht hierbei aus üblichen synthetischen oder halbsynthetischen Fasern, wie Polyestern, Polyolefinen und Rayon, oder aus üblichen natürlichen Fasern, wie Baumwolle. Bei nichtgewebten Materialien sind die Fasern in der Regel durch Bindemittel wie Polyacrylate zu verbinden. Bevorzugte Materialien sind Polyester, Rayon, Polyethylen und Polypropylen. Beispiele für flüssigkeitsdurchlässige Schichten werden beispielsweise in WO 99/57355 A1 und EP 1 023 883 A2 beschrieben.

Die flüssigkeitsundurchlässige untere Schicht (B) besteht üblicherweise aus einer Folie aus Polyethylen oder Polypropylen. Sie kann aber auch aus jedem anderen folienbildenden Polymer bestehen, beispielsweise aus Polyester, Polyamid, insbesondere bioabbaubare Polyester.

Die erfindungsgemäßen wasserabsorbierenden Speicherschichten sind im Wesentlichen frei von Zellulosefasern oder weisen einen Anteil an Zellulosefasern von vorzugsweise weniger als 30 Gew.-%, bevorzugt weniger als 20 Gew.-%, besonders bevorzugt weniger als 10 Gew.-%, ganz besonders bevorzugt weniger als 5 Gew.-%, auf. Die einsetzbaren wasserabsorbierenden Polymerpartikel unterliegen keiner Beschränkung. Vorzugsweise werden aber wasserabsorbierende Polymerpartikel mit einer Flüssigkeitsweiterleitung (SFC) von 50 bis 150 x 10⁻⁷cm³s/g verwendet, wobei die Flüssigkeitsweiterleitung (SFC) gemäß der in der WO 2008/092843 A1 (Seite 30, Zeilen 16 bis 36) beschriebenen Methode bestimmt werden kann.

Ebenso können wasserabsorbierende Polymerpartikel mit einer Permeabilität (GBP) von 10 bis 100 darcies verwendet werden. In einer besonderen Ausführungsform werden wasserabsorbierende Polymerpartikel mit einer Permeabilität (GBP) von 100 bis 1000 darcies verwendet. Die Permeabilität (GBP) wird gemäß US 2005/0256757 bestimmt.

Weiterhin ist es vorteilhaft wasserabsorbierende Polymerpartikel mit einer Zentrifugenretentionskapazität (CRC) von mindestens 33 g/g und einer Absorption unter Druck von 49,2 g/cm²(AUL0.7psi) von mindestens 12 g/g einzusetzen.

Es ist darüber hinaus vorteilhaft, wenn die Absorptionsgeschwindigkeit der wasserabsorbierenden Polymerpartikel für wässrige Körperflüssigkeiten den jeweiligen Anforderungen in der wasserabsorbierenden Speicherschicht optimal angepasst ist. Zur Bestimmung der Absorptionsgeschwindigkeit wird bevorzugt der in der Literatur beschriebene Vortex-Test eingesetzt, beispielsweise in der Monographie "Modern Superabsorbent Polymer Technology". F.L. Buchholz und A.T. Graham, Wiley-VCH, 1998, auf den Seiten 156 und 157. Die Vortex-Zeiten der wasserabsorbierenden Polymerpartikel sollten weniger als 120 Sekunden, vorzugsweise weniger als 80 Sekunden, bevorzugt weniger als 50 Sekunden, besonders bevorzugt weniger als 40 Sekunden, ganz besonders bevorzugt weniger als 20 Sekunden, betragen.

Die Aufnahme- und Verteilschicht (D) besteht üblicherweise aus Zellulosefasern, modifizierte Zellulose oder synthetischen Fasern und hat die Aufgabe wässrige Flüssigkeiten, beispielsweise Urin, schnell aufzunehmen und an die wasserabsorbierende Schicht (C) weiterzuleiten.

Für die Aufnahme- und Verteilschicht (D) werden vorzugsweise modifizierte, besonders bevorzugt chemisch modifizierte, ganz besonders bevorzugt chemisch versteifte, Zellulosefasern verwendet. Geeignete Mittel zur chemischen Versteifung sind kationisch modifizierte Stärke, Polyamid-Epichlorhydrin-Harze, Polyacrylamide, Harnstoff-Formaldehyd-Harze, Melamin-Formaldehyd-Harze und Polyethyleniminharze.

Die Versteifung kann auch durch Veränderung der chemischen Struktur erfolgen, beispielsweise durch Vernetzung. Die Vernetzer können die Polymerketten durch Ausbildung kovalenter Bindungen vernetzen. Geeignete Vernetzer sind beispielsweise C₂- bis C₈-Dialdehyde, C₂- bis C₈-Monoaldehyde mit einer Carbonsäuregruppe und C₂- bis C₈-Dicarbonsäuren.

Gemäß der vorliegenden Erfindung werden verbesserte wasserabsorbierende Speicherschichten erhalten, sowie Hygieneartikel, die diese enthalten. Durch die Trennung von Flüssigkeitsspeicherung und Flüssigkeitsleitung kann hierbei einerseits der Materialverbrauch, insbesondere an Fasern, zur Herstellung dieser Speicherschichten deutlich gesenkt werden, andererseits werden dünne und weiche Hygieneartikel erhalten, die trocken und im Gebrauch eine hervorragende Integrität aufweisen, da die wasserabsorbierenden Polymerpartikel wesentlich effizienter fixiert werden können.

## Patentansprüche

1. Wasserabsorbierende Speicherschicht bestehend aus einer Vliesunterlage (12), wasserabsorbierenden Polymerpartikeln und einer flüssigkeitsdurchlässigen Abdeckung (13), **dadurch gekennzeichnet, dass** sich zwischen der Vliesunterlage und der flüssigkeitsdurchlässigen Abdeckung eine flüssigkeitsdurchlässige Matrix (15) befindet und die wasserabsorbierenden Polymerpartikel (14) in die Kammern der Matrix eingebracht wurden.

2. Wasserabsorbierende Speicherschicht gemäß Anspruch 1, **dadurch gekennzeichnet, dass** die flüssigkeitsdurchlässige Matrix mit der Vliesunterlage und der flüssigkeitsdurchlässigen Abdeckung verklebt wurde.

3. Wasserabsorbierende Speicherschicht gemäß einem der Ansprüche 1 bis 2, **dadurch gekennzeichnet, dass** es sich bei der flüssigkeitsdurchlässigen Matrix um verpresste hydrophile Fasern oder offenporige weiche Schwämme handelt.

4. Wasserabsorbierende Speicherschicht gemäß einem der Ansprüche 1 bis 3, **dadurch gekennzeichnet, dass** die flüssigkeitsdurchlässige Matrix im expandierten Zustand durchgehende Poren mit 0,001 bis 2,0 mm Durchmesser aufweist.

5. Speicherschicht gemäß einem der Ansprüche 1 bis 4, wobei die wasserabsorbierenden Polymerpartikel eine Zentrifugenretentionskapazität von mindestens 15 g/g aufweisen.

6. Hygieneartikel, enthaltend eine wasserabsorbierende Speicherschicht gemäß einem der Ansprüche 1 bis 5.

## Claims

1. A water-absorbing storage layer consisting of a nonwoven backsheet (12), water-absorbing polymer particles and a liquid-permeable topsheet (13), wherein a liquid-permeable matrix (15) is present between the nonwoven backsheet and the liquid-permeable topsheet, and the water-absorbing polymer particles (14) have been introduced into the chambers of the matrix.

2. The water-absorbing storage layer according to claim 1, wherein the liquid-permeable matrix has been adhesive bonded to the nonwoven backsheet and the liquid-permeable topsheet.

3. The water-absorbing storage layer according to either of claims 1 and 2, wherein the liquid-permeable matrix comprises pressed hydrophilic fibers or open-pore soft sponges.

4. The water-absorbing storage layer according to any of claims 1 to 3, wherein the liquid-permeable matrix in the expanded state has continuous pores with diameter 0.001 to 2.0 mm.

5. The storage layer according to any of claims 1 to 4, wherein the water-absorbing polymer particles have a centrifuge retention capacity of at least 15 g/g.

6. A hygiene article comprising a water-absorbing storage layer according to any of claims 1 to 5.

## Revendications

1. Couche de rétention absorbant l'eau, consistant en un support en non-tissé (12), des particules de polymère absorbant l'eau et un recouvrement (13) perméable aux liquides, **caractérisée en ce qu'**entre le support en non-tissé et le recouvrement perméable aux liquides se trouve une matrice (15) perméable aux liquides et les particules de polymère (14) absorbant l'eau ont été introduites dans les chambres de la matrice.

2. Couche de rétention absorbant l'eau selon la revendication 1, **caractérisée en ce que** la matrice perméable aux liquides a été collée au support en non-tissé et au recouvrement perméable aux liquides.

3. Couche de rétention absorbant l'eau selon l'une quelconque des revendications 1 et 2, **caractérisée en ce que** pour ce qui est de la matrice perméable aux liquides il s'agit de fibres hydrophiles pressées ou d'éponges souples à pores ouverts.

4. Couche de rétention absorbant l'eau selon l'une quelconque des revendications 1 à 3, **caractérisée en ce que** la matrice perméable aux liquides présente à l'état expansé des pores intercommunicants de 0,001 à 2,0 mm de diamètre.

5. Couche de rétention selon l'une quelconque des revendications 1 à 4, dans laquelle les particules de polymère absorbant l'eau présentent un pouvoir de rétention centrifuge d'au moins 15 g/g.

6. Article d'hygiène, contenant une couche de rétention absorbant l'eau selon l'une quelconque des revendications 1 à 5.
